# EUROPEAN PATENT APPLICATION

(11) **EP 3 792 234 A1**
(43) Date of publication of application: **17.03.2021**
(21) Application number: 19306114.0
(22) Date of filing: 16.09.2019
(51) Int. Cl.: C07C 1/24, C07C 5/25, C07C 11/10, C08F 210/16

(54) **METHOD FOR THE PREPARATION OF 2-METHYL-BUT-2-ENE**

(71) Applicant: Total Raffinage Chimie, 92400 Courbevoie (FR)
(72) Inventor: MINOUX, Delphine, 1400 Nivelles (BE); RICHARDSON, Christine-Joy, King of Prussia, Pennsylvania 19406 (US); NELSON, Keith, Exton, PA 19341 (US)
(74) Representative: Fédit-Loriot

(57) **Abstract**

Method for the preparation of a composition enriched in 2-methyl-but-2-ene and use for making a polymer.

## Description

### Background

Tackifying resins belong to a class of polymers that is characterized by low molecular weight, high glass transition temperature, and a roughly linear correlation of Tg to molecular weight in polymers of similar structure. These resins are made from copolymerization of styrene or derivative, a diene such as piperylene and a C₅ olefin. Preferred C₅ olefin is chosen among specific isomers of methyl butenes aka isoamylenes, namely 2-methylbut-2-ene (2MB2) and 2-methylbut-1-ene (2MB1).

2MB2 (isoamylene) and 2MB1 are used in resin polymerization to control Tg and molecular weight due to their propensity for chain transfer. Their reactivity and chain transfer capability is a result of their branched olefin structure. 3-methylbut-1-ene (3MB1), being a terminal non-branched olefin, is less capable in this capacity.

2MB2 and 2MB1 are usually produced by the deep catalytic cracking (DCC) of vacuum gas oil. DCC is similar to fluid catalytic cracking (FCC) and produces a higher yield of propylene, isobutylene, and isoamylene. With increased demand for propylene, DCC has grown in favor. Nonetheless, one could consider alternative methods to the production of branched C₅ olefins via dehydrogenation and/or isomerization of normal olefins and alkenes and the enzymatic conversion of hydroxyalkanoic acids.

Below are references for the production of isoamylenes and other C₄ and C₅ branched olefins, and their use:
US 5221776 describes a catalyst for the isomerization of C₅ olefins to produce isoamylenes. US 5243121 describes a fluid catalytic cracking process for increased production of isobutylene and isoamylenes. US 5166455 describes a process for converting C₅-C₇ olefins (e.g. FCC light naphtha) to isobuene- and isoamylene-rich streams. WO 2012052427A1 describes the production of alkenes by the combined enzymatic conversion of 3-hydroxyalkanoic acids using different mevalonate pyrophosphate decarboxylases.

US 8378160 describes a process for preparing a hydrocarbon olefin composition from a feedstock derived from biomass. The process includes dehydrating isobutanol to obtain C₄ olefins, which are then oligomerized to form dimers and trimers.

2MB2 is most commonly used as a starting material for other products as opposed to being used as is for some final application. While not an exhaustive one, the public literature reveals several uses for isoamylene. These include (i) hydrocarbon resin modification (softening point/Tg/molecular weight control), (ii) fuel additives via oligomerization (typically dimerization) for octane boosters or via etherification with methanol or ethanol, (iii) synthetic building block such as precursor to diolefins, flavor/fragrance enhancers, antioxidants, typically alkyl phenols, or as synthon for fine chemicals or pharmaceutical ingredients preparation.

With regards to hydrocarbon Resin Modification, WO 2012050658A1 describes the use of isoamylene to control softening point and molecular weight (Mz) in the synthesis of hydrocarbon resins, US 5656698 describes use in the synthesis of hydrocarbon tackifying resins, US 4677176 also describes use in the synthesis hydrocarbon tackifying resins.

As per fuel additive, US 20120157725A1 describes the partial hydrogenation of isoprene to a mixture of isoamylenes which can be reacted with alcohols to afford oxygenates such as TAME, acid catalytically dimerized, or reacted with HF to produce high octane alkylates. Fuel Processing Technology (2015)138, 86-99 describes the use of cationic exchange resins for the oligomerization of isoamylene for production of octane boosters. Biofuels, Bioadditives & Biorefining (2014), 8(5), 658-669 describes the catalytic etherification of glycerol (a byproduct of biodiesel production) and isoamylenes to produce oxygenated fuel additives. Advanced Material Research (Durnten-Zurich, Switzerland) (2013), 805-806 describes catalysts for the etherification of isoamylene and methanol. Chemical Engineering Research and Design (2014), 92(4), 644-656 describes catalysts for the simultaneous etherification of isobutene and isoamylenes with ethanol. Renewable & Sustainable Energy Reviews (2012), 16(9), 6717-6724 is a review of methods for the etherification of glycerol with light olefins such as isobutene and isoamylenes. Fuel Processing Technology (2012), 102, 1-10 describes the synthesis of tert-amyl ethyl ether (TAEE) from isoamylene and ethanol. US 20060030741 describes a process for the etherification of C₄, C₅, and /or C₆ iso-olefins.

As synthetic precursor to diolefins building block, US 20080306320A1/US 7696395B2 (Fina Technology) describes a method for the dehydrogenation of isoamylene to make isoprene, and US 20100022817 describes the dehydrogenation of hydrocarbons to alkenes, e.g. n-pentene to piperylene, n-butane to butadiene, and isoamylene to isoprene.

As synthetic flavor and fragrance enhancer building block, US 4366078 (International Flavors and Fragrances) describes the dimerization of isoamylene to form diisoamylene mixture that is used as an aroma enhancer. US 4608193 claims isochroman derivatives made from alpha methylstyrene and isoamylene as aroma potentiators in perfumes. US 4359412 describes the production of C11 acetates used as flavoring agents via isoamylene dimerization. Reaction of the product with formaldehyde via the Prins reaction is followed by acetylation by treatment with acetic anhydride. US 4303555 describes the production of isoamylene dimers for enhancing or augmenting the aroma of perfumes or colognes. Industrial & Engineering Chemistry Research (2010), 49(12), 5549-5560 describes the cycloaddition of isoamylene and alpha methylstyrene to form 1,1,2,3,3-pentamethylindane, an intermediate in the synthesis of musk fragrances.

As antioxidant, US 3932537 describes a method for the alkylation of phenol with isobutylene or isoamylene. JP 07223985 describes the preparation of 2-methyl-6-tert-amyl phenol via reaction of cresol with isoamylene. The product is an intermediate for phenolic antioxidants. US 20100069542 describes use of amylene to synthesize liquid amylaryl phosphites that are then used as stabilizers in various polymers.

Other uses of isoamylenes are described in the following papers: Polymer Preprints (ACS, Division of Polymer Chemistry) (1999), 40(2), 786-787 discusses the use of isoamylene in the synthesis of anionic initiators. J. of Chinese Pharmaceutical Sciences (2013), 22(4), 355-360 describes the synthesis of methoxy methyl ether isoamylene quercetin (MIAQ) that are useful in assisting the healing of injured rat aorta endothelial cells. Chemical Engineering & Technology (2001), 24(3), 242-245 describes the dewatering of chloroform by the catalytic conversion of isoamylene to isoamyl alcohol.

Albeit isoamylenes are commercially available, cheaper alternative sources as well as alternative methods of making the same are desirable.

### Summary

The present invention is mostly directed to a method for the preparation of a composition enriched in 2-methyl-but-2-ene comprising the successive steps of:
(i) dehydrating a first composition comprising at least 30 wt% of a mixture of C₅ alcohol isomers to produce a first C₅ olefin mixture and water stream, wherein said first C₅ olefin mixture comprises 2-methyl-but-2-ene, 2-metyl-but-1-ene and optionally 3-methyl-but-1-ene,
(ii) removing water from the first C₅ olefin mixture,
(iii) isomerizing the first C₅ olefin mixture of step (ii) to produce the composition enriched in 2-methyl-but-2-ene.

### Detailed description

According to a first aspect, the instant invention discloses a method for the preparation of a composition enriched in 2-methyl-but-2-ene comprising the successive steps of (i) dehydrating a first composition comprising at least 30 wt% of a mixture of C₅ alcohol isomers to produce a first C₅ olefin mixture and water stream, wherein said first C₅ olefin mixture comprises 2-methyl-but-2-ene, 2-metyl-but-1-ene and optionally 3-methyl-but-1-ene, (ii) removing water from the first C₅ olefin mixture, (iii) isomerizing the first C₅ olefin mixture of step (ii) to produce the composition enriched in 2-methyl-but-2-ene.

It has been found that the above-mentioned C₅ olefin mixture could be efficiently obtained by sequential dehydration of a first composition comprising an appropriate alcohol or alcohol mixture using proper dehydration catalyst followed by isomerization. In addition, resulting C₅ olefin mixture has been found to be particularly suitable for making a polymer, in part due to enhanced content in 2-methyl-but-2-ene (2MB2).

The composition enriched in 2-methyl-but-2-ene of step (iii) is (iv) separated into (a) a second C5 olefin mixture stream and into (b) a third stream. It is particularly desirable to remove water from the first C₅ olefin mixture at step (ii).

The third stream (b) of step (iv), when containing significant amount of straight (linear) C₅ olefins is preferably partly or totally sent back to dehydration step (i). Sending back this third stream to dehydration was found advantageous since these straight C₅ olefins could be isomerized under similar reaction conditions as those used for dehydrating C₅ alcohols. Using this method allows lowering industrial unit construction expenses. Depending on operating conditions, the amount of straight (linear) C₅ olefins can be in the 5-15 wt% range.

The first composition advantageously contains at least 40 wt% of a mixture of C₅ alcohol isomers, preferably at least 70 wt%, more preferably at least 80 wt%, even more preferably at least 90wt%.

Ideally, the first composition contains an as pure as possible mixture of C₅ alcohol isomers. In this respect, it is preferred that the first composition of step (i) further comprises at most 5wt% of compounds having boiling point greater than 408K under a pressure of 101.3 kPa. It was observed that lowering the proportion of products having higher boiling point improved unit run time without failure by lowering catalyst deactivation, clogging occurrence and by products.

Similarly, but less critically, the first composition of step (i) further comprises at most 5wt% of compounds having boiling point lower than 383K under a pressure of 101.3 kPa. Withdrawal of these low boiling point products is advantageous in that it allows avoiding or at least limiting the need for separating the resulting side products issued from the process of the instant invention such as butenes or other low boiling point products.

The said first C₅ olefin mixture of step (i) preferably 2-methyl-but-2-ene, 2-metyl-but-1-ene and 3-methyl-but-1-ene, and optionally cis-2-pentene and/or trans-2-pentene.

The mixture of C₅ alcohol isomers of step (i) is preferably obtained by distillation of a fusel oil.

Dehydration step (i) is performed using a dehydration catalyst, preferably an alumina or an alumino-silicate, more preferably alumina or a zeolite. More specifically, dehydration step (i) is preferably performed using a zeolite, preferably selected among FER, ZSM-35, ZSM-22, modified or unmodified MFI, and is performed at temperature comprised from 500K to 730K, preferably from 520K to 700K, more preferably from 520K to 670K, with a LHSV comprised from 0.1 h⁻¹ to 20 h⁻¹, preferably from 1 h⁻¹ to 10 h⁻¹. Temperature range and LHSV may be adapted by those skilled in the art, based on catalyst performance and sensitivity thereof to poisons and water, and based on targeted products yields.

Isomerization step (iii) is preferably performed using an acidic catalyst, at a temperature lower than 350K, preferably lower than 320K, more preferably lower than 300K, with a LHSV comprised from 0.1 h⁻¹ to 20 h⁻¹, preferably from 1 h⁻¹ to 10 h⁻¹, and wherein the acidic catalyst is an acidic zeolite or a sulfonic acid resin, preferably a sulfonic acid resin, more preferably a sulfonic acid grafted polystyrene resin.

According to a very preferred embodiment 1, isomerization step (iii) is performed using an acidic catalyst, at a temperature lower than 350K, preferably lower than 320K, more preferably lower than 300K, with a LHSV comprised from 0.1 h⁻¹ to 20 h⁻¹, preferably from 1 h⁻¹ to 10 h⁻¹, and wherein the acidic catalyst is a sulfonic acid resin, more preferably a sulfonic acid grafted polystyrene resin, if the C₅ olefin mixture stream contains less than 20%wt linear C₅, preferably less than 15%wt and most preferably less than 8%wt linear C₅ and less than 10%wt 3MB1, preferably less than 5%wt 3MB1 and most preferably less than 3%wt.

Alternatively, and according to a very preferred embodiment 2, isomerization step (iii) is performed using a crystalline silicate catalyst, at a temperature in the range of than 523K and 850K, preferably in the range of 573K and 823K, more preferably in the range of 623K and 823K, with a LHSV comprised from 0.5 h⁻¹ to 30 h⁻¹, preferably from 1 h⁻¹ to 20 h⁻¹, if the C5 olefin mixture stream contains 3MB1 in the range of 5 to 60%wt, preferably in the range of 8 to 40%wt.

The first C₅ olefin mixture of step (ii) is separated from water using a flash drum or distillation or liquid/liquid separation to provide a water fraction and a C₅ olefin enriched fraction, and wherein the C₅ olefin fraction is optionally further dehydrated using (a) a desiccating agent such as silica gel, anhydrous sodium carbonate or anhydrous sodium sulfate, (b) a molecular sieve, (c) organic or inorganic membrane having adapted porosity, (d) optionally azeotropic distillation, (e) water trapping agents such as oleum, phosphorous pentoxide, Na₂O or CaO, preferably (b) molecular sieve or (d) optionally azeotropic distillation, or their combination.

The above-described method for the preparation of a composition enriched in 2-methyl-but-2-ene is particularly suitable for the preparation of a polymer composition, wherein the said composition enriched in 2-methyl-but-2-ene is (v) further reacted with (a) a vinyl aromatic such as 2-methylstyrene, (b) a conjugated diene such as a piperylene isomer or a mixture thereof, butadiene and/or isoprene, in the presence of (c) a lewis acid such as aluminum chloride, to obtain the said polymer composition.

In this respect, the polymer composition has advantageously a number average molecular mass Mn from 400 to 2400 g/mol, a mass average molecular mass Mw from 900 to 4000 g/mol, a Z-average molecular mass Mz from 1500 to 6000 g/mol, a molecular weight distribution Mw/Mn from 1.50 to 1.90, preferably a number average molecular mass Mn from 600 to 1400 g/mol, a mass average molecular mass Mw from 1000 to 2400 g/mol, and a Z-average molecular mass Mz from 2000 to 4000 g/mol.

The polymer composition is preferably (a) under the form of a tackifying resin, or (b) combined with an elastomer under the form of an adhesive composition.

An advantageous source of first composition comprising at least 30 wt% of a mixture of C₅ alcohol isomers is raw or refined fusel oil, preferably a C₄₊ or C₄-C₆ cut, more preferably a C₅ cut isolated from fusel oil. Raw fusel oil corresponds to the distillation bottoms of ethanol produced by fermentation of biomass, such as sugar cane, sugar beetroot, corn (maize), potatoes or any other vegetal source that is susceptible to produce alcohols by fermenting. A C₄₊ cut here corresponds to a composition essentially comprising molecules having 4 or more carbon atoms within their backbone. For instance, 1-butanol, 2-methyl-1-propanol, 3-methyl-2-butanol, ethyl-pentanoate are molecules comprising respectively 4, 4, 5 and 7 carbon atoms.

The term "essentially all", as present in this document, means more than 80% of the identified subject matter to which it refers, preferably more that 90%, more preferably more than 95%, and even more preferably more than 98% of the subject matter to which it refers. When the term "essentially all" directly refers to a product or a composition, percentage is weight percent (wt%).

Above-mentioned C₄₊ cut can be obtained by distillation of fusel oil until all or essentially all of C₃ containing products and lighter products are evaporated from the fusel oil.

Albeit not preferred, the C₅ olefin mixture may optionally contain at least one C₆ olefin chosen among 1-hexene, 2-hexene, 3-hexene, 2-methyl-1-pentene, 3-methyl-1-pentene, 4-methyl-1-pentene, 2-ethyl-1-butene, 2,3-dimethyl-1butene, 2,3-dimethyl-2butene, cyclohexene, 2-methyl-2-pentene, 3-methyl-2-pentene, 3,3-dimethyl-1-butene, 1-methyl-cyclopentene, 3-methyl-cyclopentene, 4-methyl-cyclopentene, methylene-cyclopentane.

The C₅ olefin mixture may optionally contain at least one C₄ olefin chosen among 1-butene, 2-butene, 2-methyl-1-propene.

The optionally substituted vinyl aromatic is chosen among styrene, alpha-methyl-styrene, a vinyl toluene, a vinyl xylene, a vinyl ethyl benzene, a vinyl ethyl toluene, a vinyl ethyl xylene, a vinyl isopropyl toluene, a vinyl isopropyl xylene, and their mixtures, and wherein the C₄-C₆ conjugated diene is selected from 1,3-butadiene, isoprene, piperylene, 1-methyl-cyclopentadiene, 2-methyl-cyclopentadiene, 5-methyl-cyclopentadiene, α-farnesene, β-farnesene, and their mixtures and their cis and/or trans isomers. Piperylene and β-farnesene are preferred.

More preferably, the optionally substituted vinyl aromatic is alpha-methyl-styrene, styrene, or their combination.

The catalyst or initiating system responsible for copolymerization of 2MB2 is preferably a Lewis acid, more preferably a Friedel-Crafts catalyst, advantageously chosen among metal halides, the metal being preferably chosen among B, Al, Ti and Sn, the halide being preferably chosen among fluoride, chloride and bromide, the metal halide being preferably selected among BF₃, AlCl₃, SnCl₄, TiCl₃ and TiCl₄.

The first composition comprising at least 30 wt% of a mixture of C₅ alcohol isomers is preferably obtained by fermentation of a biomass feedstock, wherein the biomass feedstock is preferably raw or refined fusel oil, more preferably a C₄₊ or C₄-C₆ cut, even more preferably a C₅ cut isolated from fusel oil.

The dehydration step of the first composition comprising at least 30 wt% of a mixture of C₅ alcohol isomers to obtain the first C₅ olefin mixture and water stream, wherein said first C₅ olefin mixture comprises 2-methyl-but-2-ene, 2-metyl-but-1-ene and optionally 3-methyl-but-1-ene C₅ olefin mixture, is carried out in the presence of a dehydration catalyst, containing at least one of (a) zeolites having preferably the MFI, MTT, FER, MEL, TON, MWW, EUO, MFS structure, (b) alumina, (c) silica-alumina, and (d) alumino silicate. Preferably, the dehydration catalyst is chosen from gamma-alumina, H-ZSM-5, H-FER, ZSM-5 containing phosphorous or any mixture thereof.

The catalyst is most preferably a ferrierite catalyst, advantageously as a zeolite ammonium ferrierite powder or as extrudate (Zeolyst, CP914 CYL-1.6).

The compositions obtained in agreement with the method of the instant invention are suitable for the preparation of tackifying resins. These tackifying resins may be starting materials for preparing e.g. adhesive composition obtained by combining tackifying resin and elastomer. In this case, the elastomer is selected from the group consisting of styrene-isoprene block copolymers, polyacrylate resins, poly ethylene vinyl acetate (EVA) resins, poly styrene butadiene resins, random styrene butadiene (SBR) copolymers, styrene butadiene block copolymers, styrene butadiene styrene (SBS) block copolymers, styrene isoprene butadiene styrene (SIBS) copolymers, styrene ethylene propylene styrene (SEPS) copolymers, styrene ethylene butylene styrene (SEBS) block copolymers, amorphous polyolefin (APO) resins, and mixtures thereof.

Unless otherwise specified in the present document, percentages (%) are given in percentage by weight based on carbon, and are noted %wt.

### Experimental

Dehydration process conditions. General procedure:
In examples 1 and 2, C₅ alcohols were dehydrated over a γ-Al₂O₃ catalyst. γ-Al₂O₃ catalyst, as pellets of 35-45 mesh (0.500-0.354 µm) is obtained by crushing γ-Al₂O₃, as 1.2 mm extrudates, which exhibit the following textural properties: specific surface area of 200 m²/g, porous distribution centered around 124 Å and porous volume of 0.588 ml/g.

A stainless-steel tubular reactor having an internal diameter of 10 mm is loaded with 20 ml of the γ-Al₂O₃ catalyst pellets. The void spaces before and after the catalyst are filled with granulated SiC of 0.5 mm.

The temperature profile is monitored with the aid of a thermocouple placed inside the reactor. Reactor temperature is increased at a rate of 60°C/h to 550°C under 45 NL/h N₂ and 10 NL/h air. Once at 550°C, nitrogen flow is then reduced to 30 NL/h. After 30 minutes, nitrogen flow is further reduced to 10 NL/h. After a further 30 minutes, nitrogen flow is stopped and airflow increased to 20 NL/h. after 1 hour, reactor temperature is then decreased to the temperature of the test and then purged by nitrogen. The nitrogen is then replaced by the C₅ alcohols feed (either a pure 3-methylbutan-1-ol feed or crude fusel oil). The catalytic tests are then performed down-flow, at near atmospheric pressure (pressure of 2 barg (bar gauge)), in a temperature range of 300-450°C and with a weight hour space velocity (WHSV) varying from 2 to 7 h⁻¹. Analysis of the products is performed by using an on-line gas chromatograph.

### Example 1: 3-methylbutan-1-ol dehydration

3-methylbutan-1-ol was fed through a pre-heater and onto the catalyst bed, with an initial internal reactor temperature of 250°C and an LHSV of 4 hr⁻¹. The temperature was then increased by 25°C at 12 h intervals until 450°C.

Complete alcohol conversion is observed from 325°C with about 86% of 3-methylbut-1-ene (kinetic isomer) 10% 2-methylbut-2-ene and 3% of 2-methylbut-1-ene. From 375°C, the proportion of 2-methylbut-2-ene (thermodynamic isomer) and/or 2-methylbut-1-ene is observed to be superior to that of 3-methylbut-1-ene. From 400°C, the proportion of 2-methylbut-2-ene is observed to be superior to that of 2-methylbut-1-ene and/or 3-methylbut-1-ene. See, table 1, below for detailed results.

**Table 1:**

| **LHSV (h⁻¹)** | 4 | 4 | 4 | 4 | 4 | 4 | 4 | 4 | 4 |
|---|---|---|---|---|---|---|---|---|---|
| **T(°C)** | 250 | 275 | 300 | 325 | 350 | 375 | 400 | 425 | 450 |
| **3MB1** | 2,5 | 5,1 | 56,6 | 86,2 | 63,3 | 38,5 | 9,3 | 4,8 | 4,9 |
| **2MB1** | 0,0 | 0,0 | 0,5 | 3,0 | 9,7 | 17,4 | 27,1 | 29,3 | 28,7 |
| **2MB2** | 0,0 | 0,1 | 2,3 | 10,0 | 26,3 | 43,1 | 62,3 | 63,8 | 60,1 |
| **Diisoamyl ether** | 76,9 | 35,8 | 13,2 | 0,1 | 0,0 | 0,0 | 0,0 | 0,0 | 0,0 |
| **3MB1-OH** | 20,2 | 58,6 | 26,5 | 0,0 | 0,0 | 0,0 | 0,0 | 0,0 | 0,0 |
| **other** | 0,4 | 0,3 | 0,9 | 0,6 | 0,7 | 1,0 | 1,3 | 2,2 | 6,4 |

| | | | | | | | | | |
|---|---|---|---|---|---|---|---|---|---|
| 3MB1: 3-methylbut-1-ene; 2MB1: 2-methylbut-1-ene; 2MB2: 2-methylbut-2-ene; 3MB1-OH: 3-methylbutan-1-ol | | | | | | | | | |

### Example 2: fusel oil dehydration

A biosourced crude fusel oil feed containing approximately 20.9 wt% ethanol, 1.5 wt% 1-propanol, 0.3 wt 1-butanol, 14.0 wt% isobutanol, 45.6 wt% 3-methylbutan-1-ol, 16.7 wt% 2-methylbutan-1-ol, 0.1 wt% ethyl pentanoate, 0.3 wt% ethyl hexanoate, and higher ethyl esters and pyrazine derivatives, is subjected to dehydration to produce C₅ olefins as main constituents.

Following filtration to remove fine particles, fusel oil was fed through a pre-heater and onto the catalyst bed, with an initial internal reactor temperature of 400°C, and an overall feed LHSV of 4 hr⁻¹. The temperature was then increased to 425°C. The results are displayed in table 2, below.

**Table 2**

| **LHSV (h⁻¹)** | 4 | 4 |
|---|---|---|
| **T (°C)** | 400 | 425 |
| **C₂ (ethylene)** | 16,0 | 10,4 |
| **C₃ (propylene)** | 1,3 | 0,9 |
| **C₄ (butenes)** | 12,2 | 7,3 |
| **3-methylbut-1-ene** | 33,2 | 11,9 |
| **2-methylbut-1-ene** | 8,9 | 7,9 |
| **2-methylbut-2-ene** | 21,0 | 17,9 |
| **Higher olefin and others** | 7,5 | 43,7 |

Complete alcohol conversion is observed at both temperatures. At 400°C, 3-methylbut-1-ene makes up around 53wt% of isoamylenes. Increasing the temperature to 425°C resulted in an increased proportion of C₅ olefin 2-methylbut-2-ene and to a decreased total isoamylenes yield due to the formation of heavier compounds.

### Example 3: distilled fusel oil dehydration

A biosourced distilled fusel oil feed (125-135°C cut) containing less than 0.1 wt% ethanol, less than 0.1 wt% 1-propanol, less than 0.1 wt% 1-butanol, approximately 1.0 wt% isobutanol, 83.5 wt% 3-methylbutan-1-ol, 13.8 wt% 2-methylbutan-1-ol, less than 0.1 wt% ethyl pentanoate, and higher ethyl esters and pyrazine derivatives, was subjected to dehydration to produce C5 olefins as main constituents, i.e. a C5 olefin mixture according to the invention.

Distilled fusel oil was fed through a pre-heater and onto the catalyst bed with an initial internal reactor temperature of 400°C, an overall feed LHSV of 4 hr⁻¹ and a pressure of 2 barg. A >99% alcohol conversion was observed with selectivity of 63% towards 2-methylbut-2-ene.

### Example 4: polymers made with C₅ olefins

Seven polymerizations of an aromatic modified aliphatic resin were completed using the various isomers of methyl butene; 2-methylbut-2-ene (2MB2), 2-methylbut-1-ene (2MB1), and 3-methylbut-1-ene (3MB1) along with their mixtures. The resulting resins were characterized by their glass transition temperatures (Tg), color, and molecular weights.

2MB2 was distilled before use. 2MB1 and 3MB1 are essentially pure and were used as purchased. In case C₅ olefins originate from fusel oil, appropriate distillation may yield desired C₅ olefins or a C₅ olefin cut. In this respect, it may be desirable for economical and/or product properties reasons to use a C₅ olefin cut comprising C₄ olefins and/or C₆ olefins.

The polymerization feed comprised piperylene concentrate which was distilled before use, alpha methylstyrene, and branched olefin. The 2MB2 in the base case blend was replaced in kind by the other branched olefins and the branched olefin blends.

The described resins were obtained by the cationic polymerization of feed blends comprising cis- and trans-piperylene, a branched olefin or branched olefin blend, and alpha-methylstyrene. They were conducted by a semi-batch mode in a round-bottom flask equipped with a stirrer and a cold water condenser. The flask was purged with nitrogen for 20 minutes before a heel of 10g of toluene was added and the reactor temperature was raised to 35° C using an external jacket. To the well-stirred toluene heel, 0.3 g of anhydrous aluminum chloride powder was added. When the powder was well dispersed, the feed blend was added at a rate of 1.5 mL/min resulting in an exothermic reaction. Subsequent 0.3 g aliquots of aluminum chloride powder were added after 10, 60, 110, and 160 mL of feed had been added over a total period of 110 minutes. When the entire feed blend (approximately 100 g) had been added, the mixture was stirred for an additional 30 minutes at which point the reaction was no longer exothermic. At this time, the catalyst was quenched with the addition of approximately 10 g of anhydrous isopropanol. The clear, yellow solution was then added to approximately 30 g of water in a 250-mL separatory funnel, shaken, and then allowed to separate into organic and aqueous phases. The lower aqueous phase was removed and the organic phase washed twice more with 25% aqueous isopropanol.

The organic phase was then transferred to a 250-mL, 3-neck flask equipped with a thermocouple, a nitrogen purge, and a Dean-Stark trap fitted with a cold water condenser, along with an antioxidant (0.2 wt% on expected resin yield). The flask was heated by means of a heating mantel to a temperature of 230° C during which time non- and un-reacted components were collected. When the pot temperature reached 230° C, the nitrogen purge was replaced with a steam purge. While maintaining a 230° C pot temperature, steam condensate was collected along with low molecular oligomeric material. When a quantity of steam condensate equal to that of resin yield (approximately 60 g), a nitrogen purge was restored in order to remove the last traces of water. The product resin was obtained as a light yellow molten liquid that solidified upon cooling to afford a clear, friable solid.

Without willing to be bound by a theory, it seems branched olefins, i.e. tri-substituted olefins, are effective chain transfer agents due to the formation of a tertiary cation when incorporated into a growing polymer chain during cationic polymerization. This tertiary cation may add another monomer or, more likely, lose a proton to a monomer thus forming an unsaturated chain end and transferring the polymerization process to a new polymer chain. Both 2MB1 and 2MB2 form more stable tertiary cations than 3MB1. One might assume that 3MB1 under the acidic conditions of Friedel Crafts polymerization, rearrange by proton migration to form 2MB2.

Results of the seven polymerizations carried out that tested the ability of the three branched olefins individually and in combination to control Tg/molecular weight in a standard tackifying formulation, are shown in Table 3, below.

Run 1 represents the base case where the only added branched olefin is 2MB2. In runs 2 and 3, 2MB1 and 3MB1 were used in place of 2MB2. Runs 4 through 7 used blends combining 2MB1, 2MB2 and 3MB1. Tg and molecular weight are generally understood to be directly related given a constant resin composition. So while 2MB1 and 2MB2 give similar tertiary cationic intermediates, they give slightly different structures upon adding another monomer (propagation) or proton loss. Polymerization of 2MB1 results in highly substituted carbons adjacent to a methylene carbon whereas polymerization product from 2MB2 contains adjacent methyl groups that hinder rotation about the common bond. Such steric hindrance is believed to increase the energy needed to achieve molecular mobility and results in a slightly higher Tg. This effect is seen in Table 3 where despite the identical molecular weights obtained in Runs 1 and 2, 2MB2 gives a resin with a higher Tg. 3MB1 is likely not as effective in chain transfer as this branched olefin produces a resin having a higher Tg and molecular weight.

Runs 4 through 7 give results that are weighted averages of the effects from the three branched olefins. For each of runs #1 to #7, piperylene, as mixture of cis + trans isomers stands for 30-35 wt% of the total feed blend, and alpha-methyl-styrene stands for 2-6 wt% of the total feed blend. The remaining is the branched olefin composition as specified in table 3, and a solvent, preferably a stream of olefins and aliphatics that is recovered by distillation from the product resin solution during finishing process.

There was no significant effect of the nature of branched olefin on resin color.

**Table 3: Physical properties**

| **Run#** | | **1** | **2** | **3** | **4** | **5** | **6** | **7** |
|---|---|---|---|---|---|---|---|---|
| **Branched olefin composition (g)** | 2MB2 | 8.5 | 0.0 | 0.0 | 2.7 | 4.7 | 5.4 | 6.0 |
| | 2MB1 | 0.0 | 8.5 | 0.0 | 1.0 | 1.2 | 2.0 | 2.0 |
| | 3MB1 | 0.0 | 0.0 | 9.9 | 4.8 | 2.6 | 1.1 | 0.6 |
| **Glass transition temperature (Tg, °C)** | | 45.5 | 38.2 | 50.8 | 48.0 | 47.5 | 45.1 | 39.8 |
| **Color, G** | | 2.4 | 2.3 | 2.4 | 2.2 | 2.3 | 2.2 | 2.3 |
| **Molecular weight** | Mn | 830 | 792 | 589 | 829 | 824 | 817 | 811 |
| | Mw | 1364 | 1312 | 1618 | 1436 | 1400 | 1361 | 1347 |
| | Mz | 2168 | 2145 | 2961 | 2403 | 2322 | 2210 | 2168 |
| **Molecular weight distribution (MWD)** | | 1.64 | 1.66 | 1.88 | 1.73 | 1.70 | 1.67 | 1.66 |
| **Conversion, %** | 2MB2 | 86.0 | 86.8 | 91.7 | 89.2 | 87.7 | 88.3 | 99.4 |
| | 2MB1 | 87.6 | 96.4 | 100.0 | 93.6 | 92.8 | 95.1 | 100.0 |
| | 3MB1 | - | - | 69.9 | 78.8 | 75.9 | 82.3 | 72.8 |

Conversion rates of 2MB1 and 2MB2 are high, generally above 85% whereas the conversion of 3MB1 is somewhat lower. This is likely due to the relatively more stable tertiary cation intermediates that are formed with 2MB1 and 2MB2 versus the less stable secondary cation formed when 3MB1 is incorporated. This is in agreement with the relatively low conversions of 2-pentenes that are generally found as a component of piperylene concentrate.

2MB1, 2MB2, and 3MB1 are each individually and in combination apt to control Tg and molecular weight in the synthesis of a typical aromatic modified resin comprised of piperylene, alpha methylstyrene, and branched olefin.

Unexpectedly, mixtures of 2MB1 and/or 2MB2 comprising significant amounts of 3MB1 were polymerized with high conversion rates, while reaching acceptable molecular weight and MWD, and achieving satisfying Tg.

First alternative dehydration process. General procedure:
In Examples 5 to 7, C₅ alcohols were dehydrated over a ferrierite catalyst. A ferrierite catalyst (Zeolyst CP914, powder) was calcined under 50 NL/h N₂ at 550°C for 6h (1°C/min). The catalyst was then crushed and sieved to 35-45 mesh. 10 mL of catalyst (5.53 g) was loaded, diluted with 10 mL of carborandum (SiC 0.5 mm).

A stainless-steel tubular reactor having an internal diameter of 10 mm is loaded with 10 mL of ferrierite catalyst. The void spaces before and after the catalyst are filled with an equivalent volume of SiC granulated at 0.5 mm. The temperature profile is monitored with the aid of a thermocouple placed inside the reactor. Analysis of the products is performed by using an on-line gas chromatograph.

Reactor temperature was increased at a rate of 60°C/h to 550°C under 10 NL/h N₂. After 1 hour, reactor temperature was then decreased to the temperature of the test and then purged by nitrogen.

### Example 5: 3-methylbutan-1-ol dehydration

3-methylbutan-1-ol was fed through a pre-heater and onto the catalyst bed, with an initial internal reactor temperature of 220°C, an LHSV of 8 hr⁻¹ and a pressure of 2 barg.

At 220°C a 3-5% alcohol conversion was observed. Increasing the temperature to 240°C resulted in only a marginal higher conversion of 4-5%. At 260°C, a 98% conversion was observed with selectivity of 59% towards 2-methylbut-2-ene. At 270°C, conversion increased to >99% and 60% 2-methylbut-2-ene. Selectivity and conversion remained constant for 60h at 270°C with no signs of catalyst deactivation.

### Example 6: 2-methylbutan-1-ol dehydration

2-methylbutan-1-ol was fed through a pre-heater and onto the catalyst bed with an initial internal reactor temperature of 240°C, an LHSV of 8 hr⁻¹ and a pressure of 2 barg. At 240°C, a 5-6% alcohol conversion was observed. At 260°C, alcohol conversion increased to 80-85% with selectivity of 59% towards 2-methylbut-2-ene.

### Example 7: distilled fusel oil dehydration

A biosourced distilled fusel oil feed (125-135°C cut) containing less than 0.1 wt% ethanol, less than 0.1 wt% 1-propanol, less than 0.1 wt% 1-butanol, approximately 1.0 wt% isobutanol, 83.5 wt% 3-methylbutan-1-ol, 13.8 wt% 2-methylbutan-1-ol, less than 0.1 wt% ethyl pentanoate, and higher ethyl esters and pyrazine derivatives, was subjected to dehydration to produce C5 olefins as main constituents, i.e. a C5 olefin mixture according to the invention.

Distilled fusel oil was fed through a pre-heater and onto the catalyst bed with an initial internal reactor temperature of 260°C, an overall feed LHSV of 8 hr⁻¹ and a pressure of 2 barg.

The temperature was then increased gradually to 375°C where a 78% isoamyl alcohol conversion was observed. At 400°C, this conversion increased to >99%, with selectivity of 55% towards 2-methylbut-2-ene. These operating conditions were maintained for 100h with no perceived loss in selectivity.

Second alternative dehydration process. General procedure:
In Examples 8 to 10, C₅ alcohols were dehydrated over a ferrierite catalyst. A ferrierite catalyst (Zeolyst, CP914 CYL-1.6) as extrudates was crushed and sieved to 35-45 mesh. 10 mL of catalyst (6.26 g) were loaded and diluted with 10 mL of carborandum (SiC 0.5 mm).

A stainless-steel tubular reactor having an internal diameter of 10 mm is loaded with 10 mL of ferrierite catalyst. The void spaces before and after the catalyst are filled with an equivalent volume of SiC granulated at 0.5 mm. The temperature profile is monitored with the aid of a thermocouple placed inside the reactor. Analysis of the products is performed by using an on-line gas chromatograph.

Reactor temperature was increased at a rate of 60°C/h to 550°C under 10 NL/h N₂. After 1 hour, reactor temperature was then decreased to the temperature of the test and then purged by nitrogen.

### Example 8: 3-methylbutan-1-ol dehydration

3-methylbutan-1-ol was fed through a pre-heater and onto the catalyst bed with an initial internal reactor temperature of 240°C, an LHSV of 8 hr⁻¹ and a pressure of 2 barg.

At 240°C, a 2-3% alcohol conversion was observed. Increasing the temperature to 250°C resulted in a higher conversion of approximately 20%. At 260°C, a >99% conversion was observed with selectivity of 60-61% towards 2-methylbut-2-ene. Selectivity remained stable for 22h at 260°C. A 3-methylbutan-1-ol feed with 8% water was then injected and the temperature maintained at 260°C for 90h during which selectivity towards 2-methylbut-2-ene remained stable at 60-61% despite formation of 1-2% heavier compounds.

### Example 9: 2-Methylbutan-1-ol dehydration

2-methylbutan-1-ol was fed through a pre-heater and onto the catalyst bed with an initial internal reactor temperature of 240°C, an LHSV of 8 hr⁻¹ and a pressure of 2 barg.

At 240°C, a 96-98% isoamyl alcohol conversion was observed towards 23-24% 2-methylbut-2-ene and 41-42% trans-2-pentene. At 250°C, the alcohol conversion increased to >99% towards approximately 50% 2-methylbut-2-ene and 24% trans-2-pentene. Increasing the reactor temperature to 260°C resulted in an increased 2MB2 selectivity around 59%. At 270°C, stable selectivity towards 60% 2MB2 was observed over 10h.

### Example 10: distilled fusel oil dehydration

A biosourced distilled fusel oil feed (125-135°C cut) containing less than 0.1 wt% ethanol, less than 0.1 wt% 1-propanol, less than 0.1 wt% 1-butanol, approximately 1.0 wt% isobutanol, 83.5 wt% 3-methylbutan-1-ol, 13.8 wt% 2-methylbutan-1-ol, less than 0.1 wt% ethyl pentanoate, and higher ethyl esters and pyrazine derivatives, was subjected to dehydration to produce C5 olefins as main constituents, i.e. a C5 olefin mixture according to the invention.

Distilled fusel oil was fed through a pre-heater and onto the catalyst bed with an initial internal reactor temperature of 270°C, and an overall feed LHSV of 8 hr⁻¹ and a pressure of 2 barg.

The temperature was then increased gradually until desired results were obtained. Initial results at 350°C showed near complete conversion (<1% alcohol) with 60% selectivity for 2MB2. Temperature increase to 360°C resulted in 62-63% selectivity, stable for 50h at 360°C (to 195h on stream). Increased temperature to 380°C appeared to marginally decrease 2MB2 selectivity in favor of trans-2-pentene and cis-2-pentene.

### Olefins isomerization

Examples 11 to 14 show different isomerization methods using various feedstocks. The acid-catalyzed isomerization of olefins via carbenium ions is well known. It may be affected by homogeneous or heterogeneous catalysis. In the following examples, we consider the use of a solid acid catalyst to increase the proportion on 2MB2 relative to 2MB1. All experiments were performed in batch but would likely be performed in a fixed bed reactor in an industrial setting.

### Example 11: Isomerization of 2MB1 to 2MB2

A 5ml vial was equipped with a stir bar was charged with 1,5 g of 2MB1 (analytical standard, Sigma Aldrich) at room temperature. 1.5 g of Amberlyst 15 (dry, hydrogen form, Sigma-Aldrich) was added to the stirred reaction vessel over approximately 1 minute and a significant exotherm was observed. Similarly, 0.1 g of Amberlyst 15 was added to 2.9 g of 2MB1. Both vials were left stirring at room temperature. Samples were taken after 1 h and 28 h and analyzed by GC-FID. Results are summarized in table 4.

**Table 4: Results of batch isomerization of 2MB1 with Amberlyst 15**

| Composition (% area) | | *3% catalyst* | | *50% catalyst* | |
|---|---|---|---|---|---|
| | Feed | 1h | 28h | 1h | 28h |
| 2MB1 | >99.5 | 11.4 | 6.2 | ∼0.1 | - |
| 2MB2 | <0.5 | 88.6 | 93.5 | ∼0.2 | - |
| Heavier compounds | n.d. | >0.05 | 0.3 | >99.5 | - |

Results showed the importance of catalyst ratio and residence time optimization in order to maximize the proportion of 2MB2 while minimizing the formation of heavier compounds.

### Example 12: Isomerization of effluent from catalytic dehydration of 3-methyl-1-butanol

The effluent from the catalytic dehydration of 3-methyl-1-butanol (table 5, below : feed) underwent the same procedure as in example 11. 3 sealed reaction vessels equipped with stir bars were charged with 32 g, 32 g and 39 g of dehydration effluent respectively at room temperature. No catalyst was added to the first reaction vessel to allow for comparison and in order to account for evaporation losses during sampling. 0.3g (∼1%) and 1.0 g (∼2.5%) of Amberlyst 15 were added to the remaining reaction vessels. Samples were taken after 1 h and 70 h and analyzed by GC-FID. Results are summarized in table 5 (results for 1% catalyst not included due to an instrument error).

**Table 5: Results of batch isomerization of a 3-methyl-1-butanol dehydration effluent with Amberlyst 15**

| Composition (%area) | | *1% catalyst* | | *2.5% catalyst* | |
|---|---|---|---|---|---|
| | Feed | 1 h | 70 h | 1 h | 70 h |
| 3MB1 | 1.97 | - | 1.95 | 1.95 | 1.91 |
| 1-pentene | 2.01 | - | 2.00 | 2.01 | 1.92 |
| 2MB1 | 18.51 | - | 4.92 | 7.59 | 3.83 |
| trans-2-pentene | 10.65 | - | 10.68 | 10.70 | 10.68 |
| cis-2-pentene | 4.81 | - | 4.78 | 4.82 | 4.68 |
| 2MB2 | 60.61 | - | 70.25 | 70.20 | 54.53 |
| Heavier compounds | 1.44 | - | 5.43 | 2.74 | 22.46 |
| ratio 2MB2/2MB1 | 77% | - | 93% | 90% | 93% |

### Example 13: Isomerization of effluent from catalytic dehydration of purified fusel oil

A test similar to example 11 was performed with the effluent from the catalytic dehydration of distilled fusel oil (Table 6: Feed) instead of 2MB1. Samples were taken after 1 h and 70 h and analyzed by GC-FID. Results are summarized in table 6.

**Table 6: Results of batch isomerization of a fusel oil dehydration effluent with Amberlyst 15**

| Composition (%area) | | 3% catalyst |
|---|---|---|
| | Feed | 1 h |
| 3MB1 | 5.12 | 5.08 |
| 1-pentene | 1.03 | 1.02 |
| 2MB1 | 24.51 | 13.16 |
| trans-2-pentene | 3.81 | 3.81 |
| cis-2-pentene | 1.91 | 1.90 |
| 2MB2 | 61.82 | 72.01 |
| Heavier compounds | 1.79 | 3.02 |
| ratio 2MB2/2MB1 | 72% | 85% |

The feed was obtained by distillation of a raw fusel oil so as to remove most of the light products such as ethanol, isobutanol, and most of the heavy compounds such as C₆₊ alcohols (e.g. 1-hexanol, 1-octanol), piperazine derivatives or residual solids. Albeit catalyst, catalyst ratio, residence time and other process conditions can be optimized, results clearly show the effectiveness of acidic catalysts for the isomerization of 2MB1 to 2MB2 in a fusel oil derived mixed C₅ olefin stream. It is also important to note that the dehydration effluent from the fusel oil feed (shown in table 6) contains lower levels of cis and trans-2-pentenes and higher levels of 2MB1 as compared to the 3-methyl-1-butanol dehydration effluent. This was a surprising result that lends itself well to the resin production process requirements and desired properties.

### Example 14: Recycling of linear pentenes over dehydration section

The dehydration of fusel oil using a ferrierite catalyst (FER) resulted in a feed as shown in table 6, containing various linear pentenes as well as 3MB1. As seen in example 13, these molecules do not isomerize under the same conditions as 2MB1.

A ferrierite catalyst (CP914 CYL-1.6, Zeolyst, extrudate) was calcined under 50 NL/h N₂ at 550°C for 6h (1°C/min). The catalyst was then crushed and sieved to 35-45 mesh. 10 mL of catalyst (5.53 g) was loaded, diluted with 10 mL of carborandum (SiC 0.5 mm).

A stainless-steel tubular reactor having an internal diameter of 10 mm is loaded with 10 mL of ferrierite catalyst. The void spaces before and after the catalyst were filled with an equivalent volume of SiC granulated at 0.5 mm. The temperature profile is monitored with the aid of a thermocouple placed inside the reactor. Analysis of the products is performed by using an on-line gas chromatograph.

Reactor temperature was increased at a rate of 60°C/h to 550°C under 10 NL/h N₂. After 1 hour, reactor temperature was then decreased to the temperature of the test and then purged by nitrogen.

3-methylbutan-1-ol was fed through a pre-heater and onto the catalyst bed, with an initial internal reactor temperature of 270°C, an LHSV of 8 hr⁻¹ and a pressure of 2 barg. After 22h, the feed was changed to a mixture composed of 90wt% of 3 methyllbutanol and 10wt % of linear pentenes under the form of a mixture of cis and trans-2-pentene. The effluent composition remained unchanged with the addition of linear pentenes to the feed.

A typical effluent composition following dehydration of fusel oil over a FER catalyst and their respective boiling points is shown in table 7.

**Table 7: typical FER effluent composition and boiling points, for feed compositions containing pure 3-methylbutan-1-ol or 3-methylbutan-1-ol in admixture with up to 10wt% of a mixture of cis/trans 2-pentene.**

| | % | Boiling point (°C) |
|---|---|---|
| 3MB1 | 2-5 | 19.7 |
| 1-C5 | 1-1.5 | 29.6 |
| 2MB1 | 23-25 | 30.8 |
| c/t 2-C5= | 5-8 | 36-36.6 |
| 2MB2 | 60-63 | 38.2 |

| | | |
|---|---|---|
| "c/t 2-C5=": cis/trans 2-pentene, (= Z/E pent-2-ene), "1-C5": 1-pentene (= pent-1-ene) | | |

Example 14 demonstrates the suitability of recycling linear pentene isomers stream in the reactor in charge of C₅ alcohol dehydration step when maximization of 2MB2 is desired. Separation of 2MB2 with linear pentenes in view of recycling of the latter may be achieved by e.g. distillation or membrane filtration.

The above examples are not limitative: Process optimization in view of industrial unit construction and operation may encompass continuous instead of batch processes as well as design optimization, including recycle loops configuration (e.g. linear pentenes recycle to dehydration reactor), among others. Similarly, the nature of the feedstock may be optimized to limit side products, operation costs or unit failure, and selection of building materials and catalysts may be adapted depending on price-to-performance ratios, industrial unit dimensions and unit operating specifications.

## Claims

1. Method for the preparation of a composition enriched in 2-methyl-but-2-ene comprising the successive steps of:
(i) dehydrating a first composition comprising at least 30 wt% of a mixture of C₅ alcohol isomers to produce a first C₅ olefin mixture and water stream, wherein said first C₅ olefin mixture comprises 2-methyl-but-2-ene, 2-metyl-but-1-ene and optionally 3-methyl-but-1-ene,
(ii) removing water from the first C₅ olefin mixture,
(iii) isomerizing the first C₅ olefin mixture of step (ii) to produce the composition enriched in 2-methyl-but-2-ene.

2. Method according to claim 1, wherein the composition enriched in 2-methyl-but-2-ene of step (iii) is (iv) separated into (a) a second C5 olefin mixture stream and into (b) a third stream.

3. Method according to claim 2, wherein the third stream of step (iv) is partly or totally sent back to dehydration step (i).

4. Method according to any one of claims 1 to 3, wherein the first composition contains at least 40 wt% of a mixture of C₅ alcohol isomers, preferably at least 70 wt%, more preferably at least 80 wt%, even more preferably at least 90wt%.

5. Method according to any one of claims 1 to 4, wherein the first composition of step (i) further comprises at most 5wt% of compounds having boiling point greater than 408K under a pressure of 101.3 kPa.

6. Method according to any one of claims 1 to 5, wherein the first composition of step (i) further comprises at most 5wt% of compounds having boiling point lower than 383K under a pressure of 101.3 kPa.

7. Method according to any one of claims 1 to 6, wherein the said first C₅ olefin mixture of step (i) comprises 2-methyl-but-2-ene, 2-metyl-but-1-ene and 3-methyl-but-1-ene, and optionally cis-2-pentene and/or trans-2-pentene.

8. Method according to any one of claims 1 to 7, wherein the mixture of C₅ alcohol isomers of step (i) is obtained by distillation of a fusel oil.

9. Method according to any one of claims 1 to 8, wherein dehydration step (i) is performed using an alumina or an alumino-silicate, preferably alumina or a zeolite.

10. Method according to any one of claims 1 to 9, wherein dehydration step (i) is performed using a zeolite, preferably selected among FER, ZSM-35, ZSM-22, modified or unmodified MFI, and wherein dehydration step (i) is performed at temperature comprised from 500K to 730K, preferably from 520K to 700K, more preferably from 520K to 670K, with a LHSV comprised from 0.1 h⁻¹ to 20 h⁻¹, preferably from 1 h⁻¹ to 10 h⁻¹.

11. Method according to any one of claims 1 to 10, wherein isomerization step (iii) is performed using an acidic catalyst, at a temperature lower than 350K, preferably lower than 320K, more preferably lower than 300K, with a LHSV comprised from 0.1 h⁻¹ to 20 h⁻¹, preferably from 1 h⁻¹ to 10 h⁻¹, and wherein the acidic catalyst is an acidic zeolite or a sulfonic acid resin, preferably a sulfonic acid resin, more preferably a sulfonic acid grafted polystyrene resin.

12. Method according to any one of claims 1 to 11, wherein the first C₅ olefin mixture of step (ii) is separated from water using a flash drum or distillation or liquid/liquid separation to provide a water fraction and a C₅ olefin enriched fraction, and wherein the C₅ olefin fraction is optionally further dehydrated using (a) a desiccating agent such as silica gel, anhydrous sodium carbonate or anhydrous sodium sulfate, (b) a molecular sieve, (c) organic or inorganic membrane having adapted porosity, (d) optionally azeotropic distillation, (e) water trapping agents such as oleum, phosphorous pentoxide, Na₂O or CaO, preferably (b) molecular sieve or (d) optionally azeotropic distillation, or their combination.

13. Use of the method for the preparation of a composition enriched in 2-methyl-but-2-ene according to any one of claims 1 to 12 for the preparation of a polymer composition, wherein the said composition enriched in 2-methyl-but-2-ene is (v) further reacted with (a) a vinyl aromatic monomer such as 2-methylstyrene, (b) a conjugated diene monomer such as a piperylene isomer or a mixture thereof, butadiene and/or isoprene, in the presence of (c) a lewis acid such as aluminum chloride, to obtain the said polymer composition.

14. The use according to claim 13, wherein the polymer composition has a number average molecular mass Mn from 400 to 2400 g/mol, a mass average molecular mass Mw from 900 to 4000 g/mol, a Z-average molecular mass Mz from 1500 to 6000 g/mol, a molecular weight distribution Mw/Mn from 1.50 to 1.90, preferably a number average molecular mass Mn from 600 to 1400 g/mol, a mass average molecular mass Mw from 1000 to 2400 g/mol, and a Z-average molecular mass Mz from 2000 to 4000 g/mol.

15. The use according to claim 13 or 14, wherein the polymer composition is (a) under the form of a tackifying resin, or (b) combined with an elastomer under the form of an adhesive composition.
